# EUROPEAN PATENT APPLICATION

(11) **EP 4 079 862 A1**
(43) Date of publication of application: **26.10.2022**
(21) Application number: 20901336.6
(22) Date of filing: 16.12.2020
(51) Int. Cl.: C12P 19/04, C12N 1/19, C12N 1/21, C12N 15/31, C12N 15/52

(54) **MICROORGANISM HAVING MODIFIED LACTOSE PERMEASE, AND METHOD FOR PRODUCING LACTOSE-CONTAINING OLIGOSACCHARIDE**

(30) Priority: 16.12.2019 JP 2019226278
(71) Applicant: Kyowa Hakko Bio Co., Ltd., Tokyo 100-8185 (JP)
(72) Inventor: TABATA Kenichiro, Tokyo 100-8185 (JP); UCHIYA Shinsuke, Tokyo 100-8185 (JP)
(74) Representative: Vossius & Partner Patentanwälte Rechtsanwälte mbB
(86) International application number: PCT/JP2020/047049
(87) International publication number: WO 2021/125245

(57) **Abstract**

An object of the invention is to provide a method for producing a lactose-containing oligosaccharide through more efficient fermentative production. According to the invention, using a microorganism having an ability of producing a protein which is modified by replacing a specific amino acid residue with a different amino acid residue and which has lactose permease activity, a lactose-containing oligosaccharide such as 2'-fucosyllactose can be produced more efficiently as compared to the case using a microorganism having an ability of producing the wild-type protein having lactose permease activity.

## Description

### TECHNICAL FIELD

The present invention relates to a method for producing a lactose-containing oligosaccharide which can produce a lactose-containing oligosaccharide highly efficiently.

### BACKGROUND ART

Milk oligosaccharides contained in human breast milk (HMOs) have been reported to have a phylactic effect against pathogenic bacteria or function as prebiotics and are receiving attention as an additive for infant formula due to the bioactivity (Non-Patent Literature 1).

So far, 130 kinds of HMO or more have been known, and most of them are lactose-containing oligosaccharides having a free lactose unit at the reducing terminal.

As a method for producing a lactose-containing oligosaccharide through fermentative production, a method of adding relatively inexpensive lactose as a substrate is known (Non-Patent Literature 2). Moreover, it is known that the uptake of lactose from outside of microbial cells is mediated by lactose permease (Non-Patent Literature 3).

However, a method for producing a lactose-containing oligosaccharide using a microorganism having a mutant lactose permease or the influence of a mutation of lactose permease on the productivity of a lactose-containing oligosaccharide is not known. At the same time, a more efficient method for producing a lactose-containing oligosaccharide is required due to the noteworthiness.

### CITATION LIST

### NON-PATENT LITERATURE

Non-Patent Literature 1: J Biotechnol (2016) 235:61-83
Non-Patent Literature 2: Curr Opin Biotechnol (2019) 56:130-137
Non-Patent Literature 3: J Biotechnol (2015) 210:107-115

### SUMMARY OF INVENTION

### TECHNICAL PROBLEM

As described above, a more efficient method for producing a lactose-containing oligosaccharide is required. An object of the invention is to provide a method for producing a lactose-containing oligosaccharide through more efficient fermentative production.

### SOLUTION TO PROBLEM

The invention relates to the followings.
1. A microorganism which has a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3] below and
   which has a higher ability of producing a lactose-containing oligosaccharide than a parent strain,
   [1] a protein consisting of the amino acid sequence represented by SEQ ID NO:2,
   [2] a mutant protein which is a protein consisting of an amino acid sequence having deletion, replacement, insertion and/or addition of 1 to 20 amino acids in the amino acid sequence represented by SEQ ID NO:2 and which has lactose permease activity, and
   [3] a homologous protein which is a protein consisting of an amino acid sequence having at least 80% identity with the amino acid sequence represented by SEQ ID NO:2 and which has lactose permease activity.
2. The microorganism according to the above 1 which has a protein consisting of an amino acid sequence containing replacement of the amino acid residue corresponding to position 319 of the amino acid sequence represented by SEQ ID NO:2 with L-aspartic acid or L-glutamic acid in the amino acid sequence of the protein described in any one of [1] to [3].
3. The microorganism according to the above 1 or 2 which has a protein consisting of an amino acid sequence containing replacement of the amino acid residue corresponding to position 319 of the amino acid sequence represented by SEQ ID NO:2 with L-glutamic acid in the amino acid sequence of the protein described in any one of [1] to [3].
4. The microorganism according to any one of the above 1 to 3 which is obtainable by transforming a parent strain with a recombinant DNA containing a DNA encoding a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3].
5. The microorganism according to the above 4 which is obtainable by incorporating the recombinant DNA containing a DNA encoding a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3] into the chromosome.
6. The microorganism according to any one of the above 1 to 5 which is obtainable by transforming a parent strain with a recombinant DNA containing a DNA having the nucleotide sequence represented by SEQ ID NO:3.
7. The microorganism according to the above 6 which is obtainable by incorporating the recombinant DNA containing a DNA having the nucleotide sequence represented by SEQ ID NO:3 into the chromosome.
8. The microorganism according to any one of the above 1 to 7, wherein the parent strain is a microorganism having an ability of producing a lactose-containing oligosaccharide.
9. A method for producing a lactose-containing oligosaccharide, comprising: culturing the microorganism according to any one of the above 1 to 8 in a medium and producing a lactose-containing oligosaccharide in a culture.
10. The production method according to the above 9, wherein the lactose-containing oligosaccharide is 2'-fucosyllactose.

### ADVANTAGEOUS EFFECTS OF INVENTION

According to the invention, a method for producing a lactose-containing oligosaccharide which can produce a lactose-containing oligosaccharide highly efficiently is provided.

### DESCRIPTION OF EMBODIMENTS

### 1. Microorganism of Invention

The microorganism of the invention is a microorganism having the protein described in (1) or (2) below and is a microorganism having a higher ability of producing a lactose-containing oligosaccharide than a parent strain.
(1) A protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3] below.
   [1] A protein consisting of the amino acid sequence represented by SEQ ID NO:2.
   [2] A mutant protein which is a protein consisting of an amino acid sequence having deletion, replacement, insertion and/or addition of 1 to 20 amino acids in the amino acid sequence represented by SEQ ID NO:2 and which has lactose permease activity.
   [3] A homologous protein which is a protein consisting of an amino acid sequence having at least 80% identity with the amino acid sequence represented by SEQ ID NO:2 and which has lactose permease activity.
(2) The protein described in (1) above which consists of an amino acid sequence containing replacement of the amino acid residue corresponding to position 319 of the amino acid sequence represented by SEQ ID NO:2 with L-glutamic acid in the amino acid sequence of the protein described in any one of [1] to [3] above.

In the amino acid sequence of the protein as the origin described in any one of [1] to [3] above, the amino acid residue corresponding to position 319 of the amino acid sequence represented by SEQ ID NO:2 refers to the amino acid residue which is aligned at the same position as the amino acid residue at position 319 of the amino acid sequence of SEQ ID NO:2 when the amino acid sequence of the original protein and the amino acid sequence of SEQ ID NO:2 are aligned.

In the amino acid sequence of the protein described in (1) or (2) above, that the amino acid residue corresponding to position 319 of the amino acid sequence represented by SEQ ID NO:2 has been replaced with a different amino acid residue can be confirmed, for example, by aligning the amino acid sequence of the protein described in (1) or (2) above, for which the amino acid residue is to be confirmed, and the amino acid sequence of the original protein described in any one of [1] to [3] above.

An alignment of amino acid sequences can be created, for example, using known alignment program ClustalW [Nucelic Acids Research 22, 4673, (1994)]. ClustalW is available, for example, at http://www.ebi.ac.uk/clustalw/ (European Bioinformatics Institute). The parameters used for creating an alignment using ClustalW can be, for example, the default values.

The different amino acid residue described in (1) above may be either a natural or unnatural amino acid residue. Natural amino acids are L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine and the like.

Examples of mutually replaceable amino acids are shown below. Amino acids included in a same group can be interchanged.
Group A: leucine, isoleucine, norleucine, valine, norvaline, alanine, 2-aminobutanoic acid, methionine, o-methylserine, t-butylglycine, t-butylalanine and cyclohexylalanine.
Group B: aspartic acid, glutamic acid, isoaspartic acid, isoglutamic acid, 2-aminoadipic acid and 2-aminosuberic acid.
Group C: asparagine and glutamine.
Group D: lysine, arginine, ornithine, 2,4-diaminobutanoic acid and 2,3-diaminopropionic acid.
Group E: proline, 3-hydroxyproline and 4-hydroxyproline.
Group F: serine, threonine and homoserine.
Group G: phenylalanine and tyrosine.

The different amino acid residue described in (1) above is preferably one selected from the amino acid residues listed in Group B above (aspartic acid, glutamic acid, isoaspartic acid, isoglutamic acid, 2-aminoadipic acid and 2-aminosuberic acid), and the amino acid residue is more preferably an L-form. The different amino acid residue described in (1) above is further preferably L-glutamic acid.

The mutant protein refers to a protein obtainable by artificially deleting or replacing an amino acid residue of the original protein or artificially inserting or adding an amino acid residue to the protein.

In the mutant protein, deletion, replacement, insertion or addition of an amino acid may be deletion, replacement, insertion or addition of 1 to 20 amino acids at any positions of a same sequence.

The deleted, replaced, inserted or added amino acids may be either natural or unnatural amino acids. Examples of the natural amino acids are the natural amino acids above.

Examples of interchangeable amino acids are as described above. Amino acids included in a same group can be interchanged.

Homologous proteins are proteins that an organism which exists in the nature has and refer to a group of proteins derived from proteins of the same evolutional origin. Homologous proteins have similar structures and functions. The identity of amino acid sequences or nucleotide sequences can be determined using the algorithm BLAST [Pro. Nat. Acad. Sci. USA, 90, 5873 (1993)] by Karlin and Altschul or FASTA [Methods Enzymol., 183, 63 (1990)]. Programs called BLASTN and BLASTX have been developed based on the algorithm BLAST [J. Mol. Biol., 215, 403 (1990)]. When nucleotide sequences are analyzed using BLASTN based on BLAST, the parameters are, for example, Score = 100 and word length = 12. When amino acid sequences are analyzed using BLASTX based on BLAST, the parameters are, for example, score = 50 and word length = 3. When the BLAST and Gap ped BLAST programs are used, the default parameters of the programs are used. Specific techniques for the analysis methods are known.

That the mutant protein or the homologous protein has lactose permease activity can be confirmed, for example, by the following method. First, a recombinant DNA having a DNA encoding the mutant protein or the homologous protein, for which the activity is to be confirmed, is produced by the following method. Next, a microorganism having no lactose permease activity, such as *Escherichia coli* strain W3110, which lacks lactose permease, is transformed with the recombinant DNA. In the end, the microorganism is cultured in a medium containing lactose as a saccharide source, and when the growth improves as compared with the parent strain, it is confirmed that the mutant protein or the homologous protein has lactose permease activity.

A specific example of the protein containing replacement of the amino acid residue corresponding to position 319 of the amino acid sequence represented by SEQ ID NO:2 with a different amino acid residue in the amino acid sequence of the protein described in (1) or (2) above is a protein consisting of the amino acid sequence represented by SEQ ID NO:32.

The parent strain refers to an original strain subjected to gene modification, transformation or the like. The original strain subjected to transformation by gene introduction is also called a host strain.

The parent strain of the microorganism of the invention may be any microorganism as long as the microorganism has an ability of producing a lactose-containing oligosaccharide.

The lactose-containing oligosaccharide refers to an oligosaccharide having a lactose unit at the reducing terminal. Examples of the lactose-containing oligosaccharide include 2'-fucosyllactose, 3-fucosyllactose, 2',3-difucosyllactose, 3'-sialyllactose, 6'-sialyllactose, 3'-sialyl-3-fucosyllactose and the like.

As the microorganism having an ability of producing a lactose-containing oligosaccharide, a breeding strain to which an ability of producing a lactose-containing oligosaccharide has been artificially given or in which the ability has been enhanced can be preferably used.

Methods for artificially giving an ability of producing a lactose-containing oligosaccharide to the microorganism used as the parent strain or for enhancing the ability in the microorganism are: (a) a method of enhancing the expression of at least one enzyme involved in the biosynthetic pathway for generating the lactose-containing oligosaccharide from a sugar; (b) a method of increasing the number of copies of at least one gene encoding an enzyme involved in the biosynthetic pathway for generating the lactose-containing oligosaccharide from a sugar; (c) a method of relieving or canceling at least one mechanism of controlling the biosynthetic pathway for generating the lactose-containing oligosaccharide from a sugar; (d) a method of weakening or blocking at least one metabolic pathway branching from the biosynthetic pathway for generating the lactose-containing oligosaccharide from a sugar into a metabolite other than the target substance; and another method. The known methods can be used singly or in combination.

Specific examples of the enzyme involved in the biosynthetic pathway for generating the lactose-containing oligosaccharide from a sugar include known enzymes such as an enzyme having α1,2-fucosyltransferase activity for generating 2'-fucosyllactose using GDP-fucose and lactose as substrates, an enzyme having α1,3-fucosyltransferase activity for generating 3-fucosyllactose using GDP-fucose and lactose as substrates, an enzyme having α2,3-sialyltransferase activity for generating 3'-sialyllactose using CMP-sialic acid and lactose as substrates and an enzyme having α2,6-sialyltransferase activity for generating 6'-sialyllactose using CMP-sialic acid and lactose as substrates.

Specific examples of the method for giving or enhancing an ability of producing a lactose-containing oligosaccharide include known methods such as a method for enhancing the ability of producing 2'-fucosyllactose or 3-fucosyllactose by genetic engineering (Metabolic Engineering (2017) 41:23-38).

A breeding strain obtained by artificially giving an ability of supplying lactose as the precursor to a microorganism having an ability of producing a lactose-containing oligosaccharide or by enhancing the ability in the microorganism can also be used.

Methods for artificially giving an ability of supplying lactose from a sugar to the microorganism used as the parent strain or for enhancing the ability in the microorganism are: (a) a method of relieving or canceling at least one mechanism of controlling the biosynthetic pathway for generating lactose from a sugar; (b) a method of enhancing the expression of at least one enzyme involved in the biosynthetic pathway for generating lactose from a sugar; (c) a method of increasing the number of copies of at least one gene encoding an enzyme involved in the biosynthetic pathway for generating lactose from a sugar; (d) a method of relieving or canceling at least one mechanism of decomposing lactose; (e) a method of weakening or blocking at least one metabolic pathway branching from the biosynthetic pathway for generating lactose from a sugar into a metabolite other than the target substance; and another method. The known methods can be used singly or in combination.

Specific examples of the enzyme involved in the biosynthetic pathway for generating lactose from a sugar include known enzymes such as an enzyme having lactose synthase activity for generating lactose using glucose and UDP-galactose as substrates.

Specific examples of the method for giving or enhancing the ability of supplying lactose include known methods such as a method of decreasing or inactivating β-galactosidase activity involved in decomposition of lactose (Metabolic Engineering (2017) 41:23-38).

The microorganism having an ability of producing a lactose-containing oligosaccharide may be any microorganism but is preferably a prokaryote or a yeast strain, more preferably a prokaryote belonging to the genus *Escherichia,* the genus *Serratia,* the genus *Bacillus,* the genus *Brevibacterium,* the genus *Corynebacterium,* the genus *Microbacterium,* the genus *Pseudomonas* or the like or a yeast strain belonging to the genus *Saccharomyces,* the genus *Schizosaccharomyces,* the genus *Kluyveromyces,* the genus *Trichosporon,* the genus *Schwanniomyces,* the genus *Pichia,* the genus *Candida* or the like, most preferably a prokaryote such as *Escherichia coli* BL21 codon plus, *Escherichia coli* XL1-Blue, *Escherichia coli* XL2-Blue (all manufactured by Agilent Technologies, Ltd.), *Escherichia coli* BL21(DE3)pLysS (manufactured by Merck Millipore), *Escherichia coli* DH5α, *Escherichia coli* HST08 Premium, *Escherichia coli* HST02, *Escherichia coli* HST04 dam⁻/dcm⁻, *Escherichia coli* JM109, *Escherichia coli* HB101, *Escherichia coli* CJ236, *Escherichia coli* BMH71-18 mutS, *Escherichia coli* MV1184, *Escherichia coli* TH2 (all manufactured by Takara Bio Inc.), *Escherichia coli* W, *Escherichia coli* JM101, *Escherichia coli* W3110, *Escherichia coli* W3110S (Kyow34, National BioResource Project), *Escherichia coli* MG1655, *Escherichia coli* DH1, *Escherichia coli* MC1000, *Escherichia coli* W1485, *Escherichia coli* MP347, *Escherichia coli* NM522, *Escherichia coli* ATCC9637, *Escherichia coli* KY3591 (deposit number: NITE BP-03062), *Serratia ficaria, Serratia fonticola, Serratia liquefaciens, Serratia marcescens, Bacillus subtilis, Bacillus amyloliquefaciens, Brevibacterium immariophilum* ATCC14068, *Brevibacterium saccharolyticum* ATCC14066, *Corynebacterium ammoniagenes, Corynebacterium glutamicum* ATCC13032, *Corynebacterium glutamicum* ATCC 14067, *Corynebacterium glutamicum* ATCC13869, *Corynebacterium acetoacidophilum* ATCC13870, *Microbacterium ammoniaphilum* ATCC15354 and *Pseudomonas* sp. D-0110 or a yeast strain such as *Saccharomyces cerevisiae, Schizosaccharomyces pombe, Kluyveromyces lactis, Trichosporon pullulans, Schwanniomyces alluvius, Pichia pastoris* and *Candida utilis.*

*Escherichia coli* KY3591 has been deposited to NITE Patent Microorganisms Depositary (NPMD), National Institute of Technology and Evaluation (NITE) located at #122, 2-5-8 Kazusakamatari, Kisarazu-shi, Chiba, Japan (postal code 292-0818). The receipt date (deposit date) is November 18, 2019, and the accession number is NITE BP-03062.

A microorganism obtained by later giving an ability of producing a lactose-containing oligosaccharide to a microorganism having the protein of (1) or (2) above produced using the host strain or by enhancing the ability in the microorganism and a microorganism obtained by further giving an ability of supplying lactose to the microorganism or by enhancing the ability in the microorganism which are microorganisms having a higher ability of producing a lactose-containing oligosaccharide than the parent strain are also the microorganisms of the invention.

Examples of the microorganism having the protein described in (1) or (2) above include a microorganism obtained by transforming a parent strain with a recombinant DNA having the DNA described in any one of (3) to (6) below and a microorganism obtained by incorporating the recombinant DNA into the chromosome.
(3) A DNA encoding the protein described in (1) or (2) above.
(4) A DNA encoding a protein consisting of an amino acid sequence containing replacement of the amino acid residue corresponding to position 319 of the amino acid sequence represented by SEQ ID NO:2 with a different amino acid residue in the amino acid sequence of a protein encoded by the DNA described in any one of [4] to [6] below.
[4] A DNA having the nucleotide sequence represented by SEQ ID NO:1.
[5] A DNA which hybridizes with a DNA consisting of a nucleotide sequence complementary to the nucleotide sequence represented by SEQ ID NO:1 under stringent conditions and which encodes a homologous protein having lactose permease activity.
[6] A DNA which consists of a nucleotide sequence having at least 95%, preferably at least 97%, further preferably at least 98%, most preferably at least 99% identity with the nucleotide sequence represented by SEQ ID NO:1 and which encodes a homologous protein having lactose permease activity.
(5) The DNA described in (4) above which encodes a protein consisting of an amino acid sequence containing replacement of the amino acid residue corresponding to position 319 of the amino acid sequence represented by SEQ ID NO:2 with L-glutamic acid in the encoded amino acid sequence.
(6) A DNA having the nucleotide sequence represented by SEQ ID NO:3.

To hybridize used above is a step in which a DNA hybridizes with a DNA having a specific nucleotide sequence or a part of the DNA. Therefore, the nucleotide sequence of the DNA which hybridizes with the DNA having a specific nucleotide sequence or a part of the DNA may be a DNA having a length which is useful as a probe for Northern or Southern blotting analysis or which can be used as an oligonucleotide primer for PCR analysis.

An example of the DNA used as a probe is a DNA of at least 100 bases or more, preferably 200 bases or more, more preferably 500 bases or more. An example of the DNA used as a primer is a DNA of at least 10 bases or more, preferably 15 bases or more.

A method for DNA hybridization experiment is well known, and the experiment can be performed by, for example, determining the conditions for hybridization according to Molecular Cloning, 4th edition (Cold Spring Harbor Laboratory Press (2012)), Methods for General and Molecular Bacteriology (ASM Press (1994)) and Immunology methods manual (Academic press (1997)) as well as many other standard textbooks.

Further, also according to an instructional manual accompanying a commercially available hybridization kit, a DNA which hybridizes under stringent conditions can be obtained. An example of the commercially available hybridization kit is random primed DNA labeling kit (manufactured by Roche Diagnostics GmbH), in which a probe is produced by the random prime method and in which hybridization is performed under stringent conditions.

The stringent conditions are, for example, conditions in which a filter on which a DNA has been immobilized and a probe DNA are incubated overnight at 42°C in a solution containing 50% formamide, 5 × SSC (750 mM sodium chloride and 75 mM sodium citrate), 50 mM sodium phosphate (pH 7.6), 5 × Denhardt's solution, 10% dextran sulfate and 20 µg/L denatured salmon sperm DNA and in which the filter is then washed, for example, in a 0.2 × SSC solution at about 65°C.

The various conditions can also be set by adding or changing a blocking reagent used for suppressing the background in the hybridization experiment. The addition of the blocking reagent may be accompanied by a change in hybridization conditions for adapting the conditions.

An example of the DNA which can hybridize under the stringent conditions is a DNA consisting of a nucleotide sequence having at least 95%, preferably at least 97%, further preferably at least 98%, most preferably at least 99% identity with the nucleotide sequence represented by SEQ ID NO: 1 when performing calculation based on the parameters using the program such as BLAST or FASTA described above.

The microorganism obtained by transforming a parent strain with a recombinant DNA having the DNA described in any one of (3) to (6) above and the microorganism obtained by incorporating the recombinant DNA into the chromosome can be created by the following methods.

The DNA described in any one of (3) to (6) above can be obtained, for example, using a DNA encoding a protein consisting of the amino acid sequence represented by SEQ ID NO:2, by introducing a mutation into the nucleotide sequence of the part encoding the amino acid residue corresponding to position 319 of the amino acid sequence represented by SEQ ID NO:2 located on the DNA by a site-specific mutagenesis method described, for example, in Molecular Cloning, 4th edition (Cold Spring Harbor Laboratory Press (2012)), Current Protocols in Molecular Biology (JOHN WILEY & SONS, INC.) or the like and thus replacing the nucleotide sequence with a nucleotide sequence encoding any amino acid residue. Alternatively, the DNA of the invention can be obtained also using PrimeSTAR Mutagenesis Basal Kit (manufactured by Takara Bio Inc.).

The DNA can also be obtained by a similar method using a DNA encoding a mutant protein which consists of an amino acid sequence having deletion, replacement, insertion and/or addition of 1 to 20 amino acids in the amino acid sequence represented by SEQ ID NO:2 and which has lactose permease activity, by introducing a mutation into the nucleotide sequence of the part, in the amino acid sequence of the mutant protein, encoding the amino acid residue which corresponds to position 319 of the amino acid sequence represented by SEQ ID NO:2 when the amino acid sequence represented by SEQ ID NO:2 and the amino acid sequence of the mutant protein are aligned by the above method.

The DNA can also be obtained using a DNA encoding a homologous protein which has an amino acid sequence having at least 80% identity with the amino acid sequence represented by SEQ ID NO:2 and which has lactose permease activity, by introducing a mutation into the nucleotide sequence of the part, in the amino acid sequence of the homologous protein, encoding the amino acid residue which corresponds to position 319 of the amino acid sequence represented by SEQ ID NO:2 when the amino acid sequence of the homologous protein and the amino acid sequence represented by SEQ ID NO:2 are aligned by the above method.

The DNA encoding a protein consisting of the amino acid sequence represented by SEQ ID NO:2 can be obtained, for example, by Southern hybridization for a chromosomal DNA library of a microorganism, preferably the genus *Escherichia,* more preferably *Escherichia coli* strain W3110 using a probe which can be designed based on the nucleotide sequence of the DNA encoding a protein consisting of the amino acid sequence represented by SEQ ID NO:2 or by PCR [PCR Protocols, Academic Press (1990)] using primer DNAs which can be designed based on the DNA encoding a protein consisting of the amino acid sequence represented by SEQ ID NO:2 and using the chromosomal DNA of *Escherichia coli* strain W3110 as a template.

*Escherichia coli* strain W3110 (ATCC27325) can be acquired from American Type Culture Collection (ATCC).

An example of the DNA encoding a protein consisting of the amino acid sequence represented by SEQ ID NO:2 is a DNA consisting of the nucleotide sequence represented by SEQ ID NO:1.

The DNA encoding the mutant protein which consists of an amino acid sequence having deletion, replacement, insertion and/or addition of 1 to 20 amino acids in the amino acid sequence represented by SEQ ID NO:2 and which has lactose permease activity described in [2] above can be obtained, for example, by subjecting a DNA consisting of the nucleotide sequence represented by SEQ ID NO:1 as a template to Error-Prone PCR or the like.

Alternatively, also by PCR using a set of PCR primers having nucleotide sequences designed to introduce the target mutation (deletion, replacement, insertion or addition) at the 5' ends [Gene, 77, 51 (1989)], the DNA encoding the mutant protein which has an amino acid sequence having deletion, replacement, insertion or addition of 1 to 20 amino acids in the amino acid sequence represented by SEQ ID NO:2 and which has lactose permease activity of [2] above can be obtained.

Further, the DNA can be obtained also according to an instructional manual accompanying a commercially available site-specific mutagenesis kit. An example of the commercially available site-specific mutagenesis kit is PrimeSTAR (registered trademark) Mutagenesis Basal Kit (manufactured by Takara Bio Inc.), which can introduce a mutation (deletion, replacement, insertion or addition) to the site to which the target mutation is to be introduced.

That is, first, a set of mutagenesis primers in which the 15 bases at the 5' ends overlap each other are designed using a plasmid having a nucleotide sequence designed to introduce the target mutation (deletion, replacement, insertion or addition) as a template. At this point, the overlapped part includes the target mutation. Next, PCR is performed using the mutagenesis primers and using a plasmid having a nucleotide sequence to which the target mutation is to be introduced as a template. When *Escherichia coli* is transformed with the amplified fragment thus obtained, a plasmid having the nucleotide sequence having the target mutation can be obtained.

The DNA encoding the homologous protein which has an amino acid sequence having at least 80% identity with the amino acid sequence represented by SEQ ID NO:2 and which has lactose permease activity described in [3] above can be obtained by, for example, searching various gene sequence databases for a nucleotide sequence having at least 80%, preferably at least 90%, more preferably at least 95%, most preferably at least 99% identity with the nucleotide sequence represented by SEQ ID NO:2 and performing a similar method to the method for obtaining the DNA encoding a protein consisting of the amino acid sequence represented by SEQ ID NO:2 using a probe DNA or primer DNAs which can be designed based on the nucleotide sequence or the amino acid sequence obtained by the search and using a microorganism having the DNA. The identity of nucleotide sequences or amino acid sequences can be determined by a similar method to that described above.

An example of the recombinant DNA having the DNA described in any one of (3) to (6) above is a recombinant DNA that is a DNA in which the DNA can autonomously replicate in the parent strain and that has the DNA described in any one or more of (3) to (6) above incorporated in an expression vector including a promoter at a position allowing the transcription of the DNA described in any one of (3) to (6) above.

The recombinant DNA described in any one of (3) to (6) above which is a DNA that can be incorporated into the chromosome in the parent strain is also the recombinant DNA having the DNA described in any one of (3) to (6) above.

When the recombinant DNA is a recombinant DNA which can be incorporated into the chromosome, a promoter does not have to be included.

When a prokaryote such as a bacterium is used as the host strain, the recombinant DNA which can autonomously replicate in the parent strain is preferably a recombinant DNA composed of a promoter, a ribosome binding sequence, the DNA described in any one or more of (3) to (6) above and a terminator sequence. A gene which controls the promoter may be included.

The distance between the Shine-Dalgarno sequence, which is a ribosome binding sequence, and the start codon is preferably adjusted to an appropriate distance, for example, to 6 to 18 bases.

In the recombinant DNA which can autonomously replicate in the parent strain, a terminator sequence is not necessarily required for the expression of the DNA, but a terminator sequence is preferably located just below the structural gene.

When a microorganism belonging to the genus *Escherichia* is used as the parent strain, examples of the expression vector include pColdI, pSTV28, pUC118 (all manufactured by Takara Bio Inc.), pET21a, pCDF-1b, pRSF-1b (all manufactured by Merck Millipore), pMAL-c5x (manufactured by New England Biolabs Inc.), pGEX-4T-1, pTrc99A (both manufactured by GE Healthcare Bio-Sciences), pTrcHis, pSE280 (both manufactured by Thermo Fisher Scientific), pGEMEX-1 (manufactured by Promega Corporation), pQE-30, pQE-60, pQE80L (all manufactured by Qiagen), pET-3, pBluescriptII SK(+), pBluescriptII KS(-) (all manufactured by Agilent Technologies, Ltd.), pKYP10 (JP-A-S58-110600), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pTrS30 [prepared from *Escherichia coli* JM109/pTrS30 (FERM BP-5407)], pTrS32 [prepared from *Escherichia coli* JM109/pTrS32 (FERM BP-5408)], pTK31 [APPLIED AND ENVIRONMENTAL MICROBIOLOGY, 2007, Vol.73, No.20, p6378-6385], pPE167 (Appl. Environ. Microbiol. 2007, 73:6378-6385), pPAC31 (WO98/12343), pUC19 [Gene, 33, 103 (1985)], pPA1 (JP-AS63-233798) and the like.

When the expression vectors are used, the promoter may be any promoter which functions in the cells of the microorganism belonging to the genus *Escherichia,* but for example, a promoter derived from *Escherichia coli,* a phage or the like, such as *trp* promoter, *gapA* promoter, *lac* promoter, PL promoter, PR promoter and PSE promoter, can be used. Moreover, an artificially designed and modified promoter, such as a promoter in which two *trp* promoters are tandemly linked, *tac* promoter, *trc* promoter, *lac*T5 promoter, *lac*T7 promoter and *let I* promoter, can also be used.

When a coryneform bacterium is used as the parent strain, examples of the expression vector include pCG1 (JP-A-S57-134500), pCG2 (JP-A-S58-35197), pCG4 (JP-AS57-183799), pCG11 (JP-A-S57-134500), pCG116, pCE54, pCB101 (all in JP-A-S58-105999), pCE51, pCE52, pCE53 [all in Molecular and General Genetics, 196, 175 (1984)] and the like.

When the expression vectors are used, the promoter may be any promoter which functions in the cells of the coryneform bacterium, but for example, P54-6 promoter [Appl. Microbiol. Biotechnol., 53, p674-679 (2000)] can be used.

When a yeast strain is used as the parent strain, examples of the expression vector include YEp13 (ATCC37115), YEp24 (ATCC37051), YCp50 (ATCC37419), pHS19, pHS15 and the like.

When the expression vectors are used, the promoter may be any promoter which functions in the cells of the yeast strain, but examples thereof include promoters such as *PHO5* promoter, *PGK* promoter, *GAP* promoter, *ADH* promoter, *gall* promoter, *gal10* promoter, heat shock polypeptide promoter, *MFα1* promoter and *CUP1* promoter.

The microorganism obtained by transforming a parent strain with the recombinant DNA refers to a microorganism which can produce the protein encoded by the DNA through transcription of the DNA as a result of introduction of the recombinant DNA as a plasmid capable of autonomously replicating in the parent strain or incorporation of the recombinant DNA into the chromosome of the parent strain.

An example of the method for confirming that the DNA described in (3) to (6) above is transcribed and that the protein encoded by the DNA is produced is measurement of the transcription amount of the DNA by Northern blotting or of the production amount of the protein by Western blotting.

The nucleotide sequence of the obtained DNA described in (3) to (6) above can be determined by incorporating the DNA into a vector by a general method directly or after cleavage with an appropriate restriction enzyme or the like, introducing the obtained recombinant DNA into host cells and then analyzing by a generally used nucleotide sequence analysis method such as the dideoxy method [Proc. Nat. Acad. Sci., USA, 74, 5463 (1977)] or using a nucleotide sequence analyzer such as Applied Biosystems 3500 genetic analyzer and Applied Biosystems 3730DNA analyzer (both manufactured by Thermo Fisher Scientific).

Examples of the host cells which can be used for determining the nucleotide sequence of the DNA of the invention include *Escherichia coli* DH5α, *Escherichia coli* HST08 Premium, *Escherichia coli* HST02, *Escherichia coli* HST04 dam-/dcm-, *Escherichia coli* JM109, *Escherichia coli* HB101, *Escherichia coli* CJ236, *Escherichia coli* BMH71-18 mutS, *Escherichia coli* MV1184, *Escherichia coli* TH2 (all manufactured by Takara Bio Inc.), *Escherichia coli* XL1-Blue, *Escherichia coli* XL2-Blue (both manufactured by Agilent Technologies, Ltd.), *Escherichia coli* DH1, *Escherichia coli* MC1000, *Escherichia coli* W1485, *Escherichia coli* W3110, *Escherichia coli* MP347, *Escherichia coli* NM522 and the like.

The vector is pBluescriptII KS(+), pPCR-Script Amp SK(+) (both manufactured by Agilent Technologies, Ltd.), pT7Blue (manufactured by Merck Millipore), pCRII (manufactured by Thermo Fisher Scientific), pCR-TRAP (manufactured by GenHunter Corporation), pDIRECT [Nucleic Acids Res., 18, 6069 (1990)] or the like.

As the method for introducing the recombinant DNA, any method for introducing a DNA into host cells can be used, and examples thereof include the method using calcium ions [Proc. Natl. Acad. Sci., USA, 69, 2110 (1972)], the protoplast method (JP-A-S63-248394), the electroporation method [Nucleic Acids Res., 16, 6127 (1988)] and the like.

When the obtained DNA is a partial length DNA as a result of the nucleotide sequence determination, the full-length DNA can be obtained by Southern hybridization method for a chromosomal DNA library using the partial length DNA as a probe or the like.

Furthermore, the target DNA can also be prepared by chemical synthesis based on the determined nucleotide sequence of the DNA using 8905-type DNA synthesizer manufactured by PerSeptive Biosystems, Inc. or the like.

Here, by substituting a base of the nucleotide sequence of the DNA described in (3) to (6) above to achieve an optimum codon for the expression in the parent strain, the expression level of the protein encoded by the DNA can also be increased. The information on the codon usage frequency of the host cells can be obtained through public database.

By inserting the DNA fragment prepared in the above manner in the downstream of the promoter of the appropriate expression vector, the recombinant DNA that the microorganism of the invention has can be produced.

An example of such a recombinant DNA is pYHA2, which is described below in the Examples.

Examples of the method for introducing the recombinant DNA as a plasmid capable of autonomously replicating in the parent strain include methods such as the method using calcium ions, the protoplast method and the electroporation method.

An example of the method for incorporating the recombinant DNA into the chromosome of the parent strain is a homologous recombination method. An example of the homologous recombination method is a method using a homologous recombination plasmid which can be produced by linking to a plasmid DNA having a drug-resistant gene which cannot autonomously replicate in the host cells for introduction. Moreover, an example of the method using homologous recombination which is often used for *Escherichia coli* is a method for introducing a recombinant DNA using the homologous recombination system of lambda phage [Proc. Natl. Acad. Sci. USA, 97, 6641-6645 (2000)].

Furthermore, using a selection method using the phenomenon that *Escherichia coli* becomes sucrose-sensitive due to levansucrase of *Bacillus subtilis* incorporated into the chromosome together with the recombinant DNA, a selection method using the phenomenon that *Escherichia coli* becomes streptomycin-sensitive by incorporation of wild-type *rpsL* gene into *Escherichia coli* having streptomycin-resistant mutant *rpsL* gene [Mol. Microbiol., 55, 137 (2005), Biosci. Biotechnol. Biochem., 71, 2905 (2007)] or the like, *Escherichia coli* in which the target region on the chromosomal DNA of the host cells has been replaced with the recombinant DNA can be obtained.

That the microorganism created by the above method is a microorganism having a higher ability of producing a lactose-containing oligosaccharide than the parent strain can be confirmed, for example, by culturing the parent strain and the created microorganism in a medium and comparing the production amounts of the lactose-containing oligosaccharide. An example of such a microorganism is strain KFL/pYHA2, which is described below in the Examples.

### 2. Production Method of Fucose-Containing Oligosaccharide of Invention

The method for producing a fucose-containing oligosaccharide of the invention is a method for producing a fucose-containing oligosaccharide characterized by culturing the microorganism of 1 above in a medium and producing a fucose-containing oligosaccharide in a culture.

The method for culturing the microorganism of 1 above can be conducted according to a general method used for culturing a microorganism. As the medium for culturing the microorganism, both a natural medium and a synthetic medium may be used as long as the medium contains a carbon source, a nitrogen source, an inorganic salt or the like which the microorganism can assimilate and as long as the microorganism can be cultured efficiently in the medium.

The carbon source may be a carbon source which the microorganism can assimilate, and for example, sugars such as glucose, fructose, sucrose, molasses containing such sugar, starch and starch hydrolysate, organic acids such as acetic acid and propionic acid, alcohols such as glycerol, ethanol and propanol and the like can be used.

As the nitrogen source, for example, ammonia, ammonium salts of inorganic acids or organic acids such as ammonium chloride, ammonium sulfate, ammonium acetate and ammonium phosphate and other nitrogen-containing compounds as well as peptone, meat extract, yeast extract, corn steep liquor, casein hydrolysate, soybean meal, soybean meal hydrolysate, fermentation bacteria, digested products thereof and the like can be used.

Examples of the inorganic salt include monopotassium phosphate, dipotassium phosphate, magnesium phosphate, magnesium sulfate, sodium chloride, ferrous sulfate, manganese sulfate, copper sulfate, calcium carbonate and the like.

The culturing is generally performed preferably under aerobic conditions such as shaking culture and submerged spinner culture under aeration. The culture temperature is generally 15 to 40°C, and the culture period is generally 5 hours to 7 days. The pH of the culture solution during the culturing is generally maintained at 3.0 to 9.0. The pH is adjusted using an inorganic or organic acid, an alkaline solution, urea, calcium carbonate, ammonia or the like.

An antibiotic such as ampicillin and tetracycline may be added to the medium during the culturing according to the need. When a microorganism transformed with an expression vector using an inducible promoter as the promoter is cultured, an inducer may be added to the medium according to the need. For example, isopropyl-β-D-thiogalactopyranoside (IPTG) or the like may be added to the medium when a microorganism transformed with an expression vector using *lac* promoter is cultured, and indoleacrylic acid or the like may be added to the medium when a microorganism transformed with an expression vector using *trp* promoter is cultured.

During the culturing, a precursor required for the production of a lactose-containing oligosaccharide, such as lactose and N-acetyllactosamine, may be added to the medium according to the need.

By producing and accumulating a lactose-containing oligosaccharide in the culture through the culturing and collecting the lactose-containing oligosaccharide from the culture, a lactose-containing oligosaccharide can be produced.

The produced lactose-containing oligosaccharide can be analyzed by a general method using carbohydrate ion chromatography or the like. The lactose-containing oligosaccharide can be collected from the culture or a treated product of the culture by a general method using activated carbon, an ion exchange resin or the like. When the lactose-containing oligosaccharide accumulates in bacterial cells, the lactose-containing oligosaccharide can be collected, for example, by crushing the bacterial cells by ultrasonic waves or the like, removing the bacterial cells by centrifugation and collecting the lactose-containing oligosaccharide from the obtained supernatant using activated carbon, an ion exchange rein or the like.

### [Analysis Examples]

In the Examples, 2'-fucosyllactose was analyzed and quantified by the procedures shown below. A culture solution containing a microorganism after culturing was centrifuged, and the supernatant was collected. 2'-Fucosyllactose contained in the supernatant was analyzed with sugar analyzer ICS-5000 (manufactured by Thermo Fisher Scientific).

### [Analysis Conditions]

Column: CarboPAC PA1
Column temperature: 25°C
Mobile phases: (mobile phase A) water
(mobile phase B) 500 mmol/L sodium hydroxide
(mobile phase C) 300 mmol/L sodium acetate
Mixing ratio of mobile phase A, mobile phase B and mobile phase C:
(0 to 10 minute) gradient of 80:20:0 to 70:20:10
(10 to 18 minute) 70:20:10
(18 to 25 minute) 80:20:0
Flow rate: 0.8 mL/min
Detector: pulsed amperometric detector

### EXAMPLES

Details of the Examples are shown below, but the invention is not limited to the Examples.

### [Example 1] Creation of Microorganism Used for Producing 2'-Fucosyllactose (1) Acquisition of DNA Fragment Used as Marker for Gene Deletion

PCR was performed using DNAs consisting of the nucleotide sequences shown in "Primer Set" in Table 1 as primer sets and using the DNAs shown in "Template" in Table 1 as templates, and amplified DNA fragments were obtained.

**[Table 1]**

| Primer Set (SEQ ID NOs:) | Template | Amplified DNA Fragment |
|---|---|---|
| 3 and 4 | pHSG396 (manufactured by Takara Bio Inc.) | *cat* |
| 5 and 6 | Genomic DNA of *Bacillus subtilis* strain 168 | *sacB* |

The genomic DNA of *Bacillus subtilis* strain 168 was prepared by a general method. The amplified DNA fragment, *cat,* includes the region from about 200 bp upstream of *cat* gene on pHSG396 to about 50 bp downstream thereof. The amplified DNA fragment, *sacB,* includes the region from about 300 bp upstream of *sacB* gene on the genomic DNA of *Bacillus subtilis* strain 168 to about 100 bp downstream thereof.

Next, PCR was performed using the amplified DNA fragments, *cat* and *sacB,* as templates and using DNAs consisting of the nucleotide sequences represented by SEQ ID NOs:3 and 6 as a primer set, and a DNA fragment including *cat* gene and *sacB* gene (called *cat-sacB* below) was obtained.

### (2) Creation of Escherichia coli which has lost β-Galactosidase Activity, Lactose Permease Activity and Colanic Acid-Producing Activity

*Escherichia coli* in which the DNA encoding β-galactosidase (called *lacZ* gene below), the DNA encoding lactose permease (called *lacY gene* below) and the DNAs encoding colanic acid production-related proteins (called *wcaJ, wzxC, wcaK, wcaL* or *wcaM* gene below) were deleted was created by the following method. Here, *lacZ* and *lacY* (called *lacZY* below) and *wcaJ, wzxC, wcaK, wcaL* and *wcaM* (called *wcaJ-wzxC-wcaKLM* below) each form an operon on the *Escherichia coli* genome.

PCR was performed using the genomic DNA of *Escherichia coli* strain KY3591 prepared by a general method as a template and using DNAs consisting of the nucleotide sequences shown in "Primer Set" in Table 2 as primer sets, and amplified DNA fragments were obtained.

**[Table 2]**

| Primer Set (SEQ ID NOs:) | Amplified DNA Fragment | Remarks |
|---|---|---|
| 7 and 8 | *lacZ* upstream 1 | The sequences at the 5' ends of the nucleotide sequences represented by SEQ ID NOs: 3 and 7 are complementary to each other. |
| 9 and 10 | *lacY* downstream 1 | The sequences at the 5' ends of the nucleotide sequences represented by SEQ ID NOs: 6 and 9 are complementary to each other. |
| 8 and 11 | *lacZ* upstream 2 | The sequences at the 5' ends of the nucleotide sequences represented by SEQ ID NOs: 11 and 12 are complementary to each other. |
| 10 and 12 | *lacY* downstream 2 | |

Here, *lacZ* upstream 1 and *lacZ* upstream 2 include the region from the start codon of *lacZ* gene to about 900 bp upstream thereof. Moreover, *lacY* downstream 1 and *lacY* downstream 2 include the region from the stop codon of *lacY* gene to about 800 bp downstream thereof.

PCR was performed using a mixture containing *lacZ* upstream 1, *lacY* downstream 1 and *cat-sacB* fragment at equivalent molar ratios as a template and using DNAs consisting of the nucleotide sequences represented by SEQ ID NOs:8 and 10 as a primer set, and a DNA fragment consisting of a sequence in which *cat-sacB* fragment was inserted into the sequence of a region around *lacZ* and *lacY* genes (called *lacZY*::*cat-sacB* below) was obtained.

PCR was performed using a mixture containing *lacZ* upstream 2 and *lacY* downstream 2 at equivalent molar ratios as a template and using DNAs consisting of the nucleotide sequences represented by SEQ ID NOs:8 and 10 as a primer set, and a DNA fragment consisting of a sequence which did not include *lacZ* or *lacY* and in which the upstream of *lacZ* and the downstream of *lacY were* linked directly (called *ΔlacZY* below) was obtained.

By introducing *lacZY*::*cat-sacB* fragment into *Escherichia coli* strain W3110 having plasmid pKD46 including a gene encoding λ recombinase [Datsenko, K. A., Warner, B. L., Proc. Natl. Acad. Sci., USA, Vol.97, 6640-6645 (2000)] by the electroporation method, a transformant exhibiting resistance to chloramphenicol and sensitivity to sucrose (a transformant in which *lacZ* and *lacY* genes were replaced with *lacZY::cat-sac_{B})* was obtained.

By introducing *ΔlacZY* fragment into the transformant by the electroporation method, transformants exhibiting sensitivity to chloramphenicol and resistance to sucrose (transformants in which *lacZY*::*cat-sacB* was replaced with *ΔlacZY*) were obtained. A transformant exhibiting sensitivity to ampicillin (a transformant lacking pKD46) was further obtained from the transformants. The transformant was named W3110Δ*lacZY*.

In a similar manner, PCR was performed using the genomic DNA of *Escherichia coli* strain KY3591 (accession number is NITE BP-03062) as a template and using DNAs consisting of the nucleotide sequences shown in "Primer Set" in Table 3 as primer sets, and amplified DNA fragments were obtained.

**[Table 3]**

| Primer Set (SEQ ID NOs:) | Amplified DNA Fragment | Remarks |
|---|---|---|
| 13 and 14 | *wcaJ* upstream 1 | The sequences at the 5' ends of the nucleotide sequences represented by SEQ ID NOs: 3 and 13 are complementary to each other. |
| 15 and 16 | *wcaM* downstream 1 | The sequences at the 5' ends of the nucleotide sequences represented by SEQ ID NOs: 6 and 15 are complementary to each other. |
| 14 and 17 | *wcaJ* upstream 2 | The sequences at the 5' ends of the nucleotide sequences represented by SEQ ID NOs: 17 and 18 are complementary to each other. |
| 16 and 18 | *wcaM* downstream 2 | |

Here, *wcaJ* upstream 1 and *wcaJ* upstream 2 include the region from the start codon of *wcaJ* gene to about 900 bp upstream thereof. Moreover, *wcaM* downstream 1 and *wcaM* downstream 2 include the region from the stop codon of *wcaM gene* to about 800 bp downstream thereof.

PCR was performed using a mixture containing wcaJ upstream 1, *wcaM* downstream 1 and *cat-sacB* fragment at equivalent molar ratios as a template and using DNAs consisting of the nucleotide sequences represented by SEQ ID NOs:14 and 16 as a primer set, and a DNA fragment consisting of a sequence in which *cat-sacB* fragment was inserted into the sequence of a region around *wcaJ-wzxC-wcaKLM* operon (called *wcaJ-wzxC-wcaKLM*::*cat-sacB* below) was obtained.

PCR was performed using a mixture containing wcaJ upstream 2 and *wcaM* downstream 2 at equivalent molar ratios as a template and using DNAs consisting of the nucleotide sequences represented by SEQ ID NOs:14 and 16 as a primer set, and a DNA fragment consisting of a sequence which did not include *wcaJ-wzxC-wcaKLM* and in which the upstream of *wcaJ* and the downstream of *wcaM were* linked directly (called Δ*wcaJ-wzxC-wcaKLM* below) was obtained.

By introducing *wcaJ-wzxC-wcaKLM*::*cat-sacB* fragment into strain W3110Δ*lacZY* created above by the electroporation method, a transformant exhibiting resistance to chloramphenicol and sensitivity to sucrose (a transformant in which *wcaJ-wzxC-wcaKLM* was replaced with *wcaJ-wzxC-wcaKLM*::*cat-sacB*) was obtained.

By introducing Δ*wcaJM* fragment into the transformant by the electroporation method, transformants exhibiting sensitivity to chloramphenicol and resistance to sucrose (transformants in which *wcaJ-wzxC-wcaKLM*::*cat-sacB* was replaced with Δ*wcaJ-wzxC-wcaKLM*) were obtained. A transformant exhibiting sensitivity to ampicillin (a transformant lacking pKD46) was further obtained. The transformant was named strain KFL.

### (3) Preparation of Escherichia coli-Derived Lactose Permease (LacY) Expression Vector

PCR was performed using DNAs consisting of the nucleotide sequences shown in "Primer Set" in Table 4 as primer sets and the DNAs shown in "Template" in Table 4 as templates, and amplified DNA fragments were obtained.

**[Table 4]**

| Primer Set (SEQ ID NOs:) | Template | Amplified DNA Fragment |
|---|---|---|
| 19 and 20 | Genomic DNA of *Escherichia coli* strain W3110 | *lacY* |
| 2 1 and 22 | pHMFT (Japanese Patent No. 4048173) | HMFT |
| 23 and 24 | Genomic DNA of *Escherichia coli* strain W3110 | *rcsA* |

The genomic DNA of *Escherichia coli* strain W3110 was prepared by a usual method. The nucleotide sequences represented by SEQ ID NOs:2 and 3 and those represented by SEQ ID NOs:4 and 5 each include complementary sequences at the 5' ends.

PCR was performed using a mixture containing *lacY,* HMFT and *rcsA* fragments at equivalent molar ratios as a template and using DNAs consisting of the nucleotide sequences represented by SEQ ID NOs:25 and 26 as a primer set, and a DNA fragment in which the three fragments were linked (called *lacY-HMFT-rcsA* below) was obtained.

PCR was performed using DNAs consisting of the nucleotide sequences represented by SEQ ID NOs:27 and 28 as a primer set and using plasmid pPE167 (Appl. Environ. Microbiol. 2007, 73:6378-6385) as a template, and a vector fragment of about 4.4 kb was obtained. Here, the nucleotide sequences represented by SEQ ID NOs:25 and 28 and those represented by SEQ ID NOs:26 and 27 each include complementary sequences at the 5' ends.

By linking *lacY*-HMFT-*rcsA* fragment and the vector fragment obtained above using In-Fusion HD Cloning Kit (manufactured by Takara Bio Inc.), expression plasmid pYHA1 was obtained.

### (4) Creation of Mutant LacY-Expressing Microorganism

Using plasmid pYHA1 obtained in (2) above as a template and using Prime STAR Mutagenesis Basal Kit (manufactured by Takara Bio Inc.), plasmid pYHA2, in which the L-lysine residue at position 319 of the amino acid sequence of LacY was replaced with L-glutamic acid residue, was created. DNAs consisting of the nucleotide sequences represented by SEQ ID NOs:29 and 30 were used as a primer set.

By transforming strain KFL created in (2) above using plasmid pYHA1 obtained in (3) and pYHA2, strain KFL/pYHA1 and strain KFL/pYHA2 were obtained.

### [Example 2] Production of 2'-Fucosyllactose by Fermentation Method using Mutant LacY-Expressing Microorganism

Strain KFL/pYHA1 and strain KFL/pYHA2 obtained in Example 1 were cultured on a LB plate at 30°C for 24 hours, inoculated to a large test tube containing 5 mL of LB medium containing 100 mg/L kanamycin and cultured by shaking at 30°C for 16 hours. Then, 0.1 mL of the obtained culture solutions were inoculated to a large test tube containing 5 mL of a production medium containing 100 mg/L kanamycin [30 g/L glucose, 5 g/L lactose monohydrate, 2 g/L magnesium sulfate heptahydrate, 16 g/L dipotassium hydrogen phosphate, 14 g/L potassium dihydrogen phosphate, 2 g/L ammonium sulfate, 1 g/L citric acid monohydrate, 5 g/L casamino acid, 10 mg/L thiamine hydrochloride, 50 mg/L ferrous sulfate heptahydrate and 10 mg/L manganese sulfate pentahydrate (those except for glucose, lactose monohydrate and magnesium sulfate heptahydrate were adjusted to pH7.2 with an aqueous sodium hydroxide solution and then autoclaved) (aqueous solutions containing glucose, lactose monohydrate and magnesium sulfate heptahydrate were prepared separately, then autoclaved, each cooled and then mixed)] and cultured by shaking at 30°C for 30 hours.

After the completion of the culturing, the culture solutions were appropriately diluted and then centrifuged, and 2'-fucosyllactose contained in the supernatants was analyzed by HPLC. The results are shown in Table 5.

**[Table 5]**

| Bacterial Strain Name | 2'-Fucosyllactose (g/L) |
|---|---|
| KFL/pYHA1 | 0.38 |
| KFL/pYHA2 | 2.58 |

As a result, strain KFL/pYHA2 exhibited a higher property of producing 2'-fucosyllactose than strain KFL/pYHA1.

It was found from the above results that, when a microorganism having mutant LacY is used, the property of producing 2'-fucosyllactose improves as compared to a microorganism having wild-type LacY.

The invention has been explained in detail referring to specific embodiments, but it is obvious to one skilled in the art that various changes and modifications are possible without departing from the spirit and the scope of the invention. The present application is based on a Japanese patent application filed on December 16, 2019 (patent application No. 2019-226278), and the entire contents thereof are incorporated by reference. Moreover, all the references cited here are incorporated in their entirety.

### INDUSTRIAL APPLICABILITY

The invention provides a method for producing a lactose-containing oligosaccharide using a microorganism having an ability of producing a protein having modified lactose permease activity.

EP 20 901 336.6
KYOWA HAKKO BIO CO., LTD.
Vossius Ref.: AF2399 EP

様式第4 | 国際
特許手続上の微生物の寄託の
国際的承認に関するブダペスト条約
下記国際寄託当局によってブダペスト条約に
基づく規則7. 1に従い発行される
原寄託についての受託証
BUDAPEST TREATY ON THE INTERNATIONAL
RECOGNITION OF THE DEPOSIT OF MICROORGANISMS
FOR THE PURPOSES OF PATENT PROCEDURE
RECEIPT IN THE CASE OF AN ORIGINAL DEPOSIT
issued pursuant to Rule 7.1 by the
INTERNATIONAL DEPOSITARY AUTHORITY
identified at the bottom of this page

寄託者 | 氏名(名称) | 協和発酵バイオ株式会社 代表取締役 社長 南方健志 様

DEPOSITOR | Name | KYOWA HAKKO BIO CO., LTD. President & CEO TAKESHI MINAKATA

住所 | 〒 100-8185
東京都千代田区大手町一丁目6番1号

Address | 1-6-1, Ohtemachi, Chiyoda-ku, Tokyo, 100-8185, Japan

I. 微生物の表示 | IDENTIFICATION OF THE MICROORGANISM
識別の表示: Identification reference given by the
DEPOSITOR:
KY3591 | 受託番号: Accession number given by the
INTERNATIONAL DEPOSITARY AUTHORITY:
NITE BP- 03062
II. 科学的性質及び分類学上の位置 | SCIENTIFIC DESCRIPTION AND/OR PROPOSED
TAXONOMIC DESIGNATION
I 欄の微生物には、次の事項が記載されていた。: The microorganism identified under I above was accompanied by:
■ 科学的性質 a scientific description
■ 分類学上の位置 a proposed taxonomic designation
III. 受領及び受託 | RECEIPT AND ACCEPTANCE
本国際寄託当局は、 2019 年 11月 18日(原寄託日)に受領した I 欄の微生物を受託した。
This International Depositary Authority accepts the microorganism identified under I above, which was received by it
on November 18, 2019 (date of the original deposit).
IV. 移管請求の受領 | RECEIPT OF REQUEST FOR CONVERSION
本国際寄託当局は、I 欄の微生物を 年 月 日(国内寄託日)に受託しており、
ブダペスト条約に基づく国際寄託への移管請求を 年 月 日に受領した。
The microorganism identified under I above was received by this International Depositary Authority
on (date of the original deposit) and a request to convert the original deposit to a deposit under the
Budapest Treaty was received by it on (date of receipt of request for conversion).
V. 国際寄託当局 | INTERNATIONAL DEPOSITARY AUTHORITY
独立行政法人製品評価技術基盤機構 特許微生物寄託センター
名称: NITE Patent Microorganisms Depositary
Name: National Institute of Technology and Evaluation
センター長 中川 恭好
Yasuyoshi Nakagawa, Director General
住所: 〒292-0818 千葉県木更津市かずさ鎌足2-5-8 122号室
Address: #122, 2-5-8 Kazusakamatari, Kisarazu-shi, Chiba 292-0818, Japan
発行日: 2019 年 12 月 6 日
Date: December 6, 2019

<110> KYOWA HAKKO BIO CO., LTD.

<120> Microorganisms having modified lactose permease and Method for
producing lactose-containing oligosaccharides

<130> W530413

<150> JP2019-226278
<151> 2019-12-16

<160> 32

<170> PatentIn version 3.5

<210> 1
<211> 1254
<212> DNA
<213> Escherichia coli
<400> 1
atgtactatt taaaaaacac aaacttttgg atgttcggtt tattcttttt cttttacttt 60

tttatcatgg gagcctactt cccgtttttc ccgatttggc tacatgacat caaccatatc 120

agcaaaagtg atacgggtat tatttttgcc gctatttctc tgttctcgct attattccaa 180

ccgctgtttg gtctgctttc tgacaaactc gggctgcgca aatacctgct gtggattatt 240

accggcatgt tagtgatgtt tgcgccgttc tttattttta tcttcgggcc actgttacaa 300

tacaacattt tagtaggatc gattgttggt ggtatttatc taggcttttg ttttaacgcc 360

ggtgcgccag cagtagaggc atttattgag aaagtcagcc gtcgcagtaa tttcgaattt 420

ggtcgcgcgc ggatgtttgg ctgtgttggc tgggcgctgt gtgcctcgat tgtcggcatc 480

atgttcacca tcaataatca gtttgttttc tggctgggct ctggctgtgc actcatcctc 540

gccgttttac tctttttcgc caaaacggat gcgccctctt ctgccacggt tgccaatgcg 600

gtaggtgcca accattcggc atttagcctt aagctggcac tggaactgtt cagacagcca 660

aaactgtggt ttttgtcact gtatgttatt ggcgtttcct gcacctacga tgtttttgac 720

caacagtttg ctaatttctt tacttcgttc tttgctaccg gtgaacaggg tacgcgggta 780

tttggctacg taacgacaat gggcgaatta cttaacgcct cgattatgtt ctttgcgcca 840

ctgatcatta atcgcatcgg tgggaaaaac gccctgctgc tggctggcac tattatgtct 900

gtacgtatta ttggctcatc gttcgccacc tcagcgctgg aagtggttat tctgaaaacg 960

ctgcatatgt ttgaagtacc gttcctgctg gtgggctgct ttaaatatat taccagccag 1020

tttgaagtgc gtttttcagc gacgatttat ctggtctgtt tctgcttctt taagcaactg 1080

gcgatgattt ttatgtctgt actggcgggc aatatgtatg aaagcatcgg tttccagggc 1140

gcttatctgg tgctgggtct ggtggcgctg ggcttcacct taatttccgt gttcacgctt 1200

agcggccccg gcccgctttc cctgctgcgt cgtcaggtga atgaagtcgc ttaa 1254

<210> 2
<211> 417
<212> PRT
<213> Escherichia coli
<400> 2

<210> 3
<211> 1254
<212> DNA
<213> Artificial Sequence
<220>
<223> Synthetic DNA
<400> 3
atgtactatt taaaaaacac aaacttttgg atgttcggtt tattcttttt cttttacttt        60

tttatcatgg gagcctactt cccgtttttc ccgatttggc tacatgacat caaccatatc       120

agcaaaagtg atacgggtat tatttttgcc gctatttctc tgttctcgct attattccaa       180

ccgctgtttg gtctgctttc tgacaaactc gggctgcgca aatacctgct gtggattatt       240

accggcatgt tagtgatgtt tgcgccgttc tttattttta tcttcgggcc actgttacaa       300

tacaacattt tagtaggatc gattgttggt ggtatttatc taggcttttg ttttaacgcc 360

ggtgcgccag cagtagaggc atttattgag aaagtcagcc gtcgcagtaa tttcgaattt 420

ggtcgcgcgc ggatgtttgg ctgtgttggc tgggcgctgt gtgcctcgat tgtcggcatc 480

atgttcacca tcaataatca gtttgttttc tggctgggct ctggctgtgc actcatcctc 540

gccgttttac tctttttcgc caaaacggat gcgccctctt ctgccacggt tgccaatgcg 600

gtaggtgcca accattcggc atttagcctt aagctggcac tggaactgtt cagacagcca 660

aaactgtggt ttttgtcact gtatgttatt ggcgtttcct gcacctacga tgtttttgac 720

caacagtttg ctaatttctt tacttcgttc tttgctaccg gtgaacaggg tacgcgggta 780

tttggctacg taacgacaat gggcgaatta cttaacgcct cgattatgtt ctttgcgcca 840

ctgatcatta atcgcatcgg tgggaaaaac gccctgctgc tggctggcac tattatgtct 900

gtacgtatta ttggctcatc gttcgccacc tcagcgctgg aagtggttat tctgaaaacg 960

ctgcatatgt ttgaagtacc gttcctgctg gtgggctgct ttaaatatat taccagccag 1020

tttgaagtgc gtttttcagc gacgatttat ctggtctgtt tctgcttctt taagcaactg 1080

gcgatgattt ttatgtctgt actggcgggc aatatgtatg aaagcatcgg tttccagggc 1140

gcttatctgg tgctgggtct ggtggcgctg ggcttcacct taatttccgt gttcacgctt 1200

agcggccccg gcccgctttc cctgctgcgt cgtcaggtga atgaagtcgc ttaa 1254

<210> 4
<211> 20
<212> DNA
<213> Artificial Sequence
<220>
<223> Synthetic DNA
<400> 4
accaggcgtt taagggcacc 20

<210> 5
<211> 45
<212> DNA
<213> Artificial Sequence

<220>
<223> Synthetic DNA
<400> 5
gactacgggc ctaaagtcga cagaataaat aaatcctggt gtccc 45

<210> 6
<211> 28
<212> DNA
<213> Artificial Sequence
<220>
<223> Synthetic DNA
<400> 6
gtcgacttta ggcccgtagt ctgcaaat 28

<210> 7
<211> 21
<212> DNA
<213> Artificial Sequence
<220>
<223> Synthetic DNA
<400> 7
tacggttagc catttgcctg c 21

<210> 8
<211> 44
<212> DNA
<213> Artificial Sequence
<220>
<223> Synthetic DNA
<400> 8
ggtgccctta aacgcctggt agctgtttcc tgtgtgaaat tgtt 44

<210> 9
<211> 19
<212> DNA
<213> Artificial Sequence
<220> 9
<223> Synthetic DNA
<400>
tcgcgcaacg cgtcagtgg 19

<210> 10
<211> 44
<212> DNA
<213> Artificial Sequence
<220>
<223> Synthetic DNA
<400> 10
gcaggcaaat ggctaaccgt aatgaacatg tcgatgacag aaag 44

<210> 11
<211> 20
<212> DNA
<213> Artificial Sequence
<220>
<223> Synthetic DNA
<400> 11
tgcgctgtgt gtcgttgggc 20

<210> 12
<211> 45
<212> DNA
<213> Artificial Sequence

<220>
<223> Synthetic DNA
<400> 12
ttctgtcatc gacatgttca tagctgtttc ctgtgtgaaa ttgtt 45

<210> 13
<211> 23
<212> DNA
<213> Artificial Sequence
<220>
<223> Synthetic DNA
<400> 13
atgaacatgt cgatgacaga aag 23

<210> 14
<211> 42
<212> DNA
<213> Artificial Sequence
<220>
<223> Synthetic DNA
<400> 14
ggtgccctta aacgcctggt cgttgttcct gttatttgcc cc 42

<210> 15
<211> 21
<212> DNA
<213> Artificial Sequence
<220>
<223> Synthetic DNA
<400> 15
aagctggtga ttaactccgg g 21

<210> 16
<211> 44
<212> DNA
<213> Artificial Sequence
<220>
<223> Synthetic DNA
<400> 16
gcaggcaaat ggctaaccgt aatttgcgac cattcctgga aaaa 44

<210> 17
<211> 20
<212> DNA
<213> Artificial Sequence
<220>
<223> Synthetic DNA
<400> 17
gcacatagcg accaacggcc 20

<210> 18
<211> 45
<212> DNA
<213> Artificial Sequence
<220>
<223> Synthetic DNA
<400> 18
tttttccagg aatggtcgca aatcgttgtt cctgttattt gcccc 45

<210> 19
<211> 23
<212> DNA
<213> Artificial Sequence

<220>
<223> Synthetic DNA
<400> 19
atttgcgacc attcctggaa aaa                                              23

<210> 20
<211> 28
<212> DNA
<213> Artificial Sequence
<220>
<223> Synthetic DNA
<400> 20
atgtactatt taaaaaacac aaactttt                                         28

<210> 21
<211> 44
<212> DNA
<213> Artificial Sequence
<220>
<223> Synthetic DNA
<400> 21
atccattcta gacctcctta attaagcgac ttcattcacc tgac                       44

<210> 22
<211> 39
<212> DNA
<213> Artificial Sequence
<220>
<223> Synthetic DNA
<400> 22
ttaaggaggt ctagaatgga ttttaagatt gtgcaagtg                             39

<210> 23
<211> 45
<212> DNA
<213> Artificial Sequence
<220>
<223> Synthetic DNA
<400> 23
atggcatacc ctcactcaat gtcaagattt tgtctcaaat tggga                      45

<210> 24
<211> 22
<212> DNA
<213> Artificial Sequence
<220>
<223> Synthetic DNA
<400> 24
cattgagtga gggtatgcca tg                                               22

<210> 25
<211> 24
<212> DNA
<213> Artificial Sequence
<220>
<223> Synthetic DNA
<400> 25
ttagcgcatg ttgacaaaaa tacc                                             24

<210> 26
<211> 43
<212> DNA
<213> Artificial Sequence

<220>
<223> Synthetic DNA
<400> 26
gaggagaaat taaccatgta ctatttaaaa aacacaaact ttt                43

<210> 27
<211> 39
<212> DNA
<213> Artificial Sequence
<220>
<223> Synthetic DNA
<400> 27
agggcatcgg tcgacttagc gcatgttgac aaaaatacc                39

<210> 28
<211> 21
<212> DNA
<213> Artificial Sequence
<220>
<223> Synthetic DNA
<400> 28
gtcgaccgat gcccttgaga g                21

<210> 29
<211> 25
<212> DNA
<213> Artificial Sequence
<220>
<223> Synthetic DNA
<400> 29
ggttaatttc tcctctttaa tatcg                25

<210> 30
<211> 27
<212> DNA
<213> Artificial Sequence
<220>
<223> Synthetic DNA
<400> 30
cagcgtttcc agaataacca cttccag                27

<210> 31
<211> 27
<212> DNA
<213> Artificial Sequence
<220>
<223> Synthetic DNA
<400> 31
attctggaaa cgctgcatat gtttgaa                27

<210> 32
<211> 417
<212> PRT
<213> Artificial Sequence
<220>
<223> Artificial Protein
<400> 32
Met Tyr Tyr Leu Lys Asn Thr Asn Phe Trp Met Phe Gly Leu Phe Phe
1               5                   10                  15

Phe Phe Tyr Phe Phe Ile Met Gly Ala Tyr Phe Pro Phe Phe Pro Ile
            20                  25                  30

Trp Leu His Asp Ile Asn His Ile Ser Lys Ser Asp Thr Gly Ile Ile
            35                  40                  45

Phe Ala Ala Ile Ser Leu Phe Ser Leu Leu Phe Gln Pro Leu Phe Gly
        50                  55                  60

Leu Leu Ser Asp Lys Leu Gly Leu Arg Lys Tyr Leu Leu Trp Ile Ile
65                  70                  75                  80

Thr Gly Met Leu Val Met Phe Ala Pro Phe Phe Ile Phe Ile Phe Gly
                85                  90                  95

Pro Leu Leu Gln Tyr Asn Ile Leu Val Gly Ser Ile Val Gly Gly Ile
            100                 105                 110

Tyr Leu Gly Phe Cys Phe Asn Ala Gly Ala Pro Ala Val Glu Ala Phe
            115                 120                 125

Ile Glu Lys Val Ser Arg Arg Ser Asn Phe Glu Phe Gly Arg Ala Arg
    130                 135                 140

Met Phe Gly Cys Val Gly Trp Ala Leu Cys Ala Ser Ile Val Gly Ile
145                 150                 155                 160

Met Phe Thr Ile Asn Asn Gln Phe Val Phe Trp Leu Gly Ser Gly Cys
                165                 170                 175

Ala Leu Ile Leu Ala Val Leu Leu Phe Phe Ala Lys Thr Asp Ala Pro
            180                 185                 190

Ser Ser Ala Thr Val Ala Asn Ala Val Gly Ala Asn His Ser Ala Phe
            195                 200                 205

Ser Leu Lys Leu Ala Leu Glu Leu Phe Arg Gln Pro Lys Leu Trp Phe
    210                 215                 220

Leu Ser Leu Tyr Val Ile Gly Val Ser Cys Thr Tyr Asp Val Phe Asp
225                 230                 235                 240

Gln Gln Phe Ala Asn Phe Phe Thr Ser Phe Phe Ala Thr Gly Glu Gln
                245                 250                 255

Gly Thr Arg Val Phe Gly Tyr Val Thr Thr Met Gly Glu Leu Leu Asn
            260                 265                 270

Ala Ser Ile Met Phe Phe Ala Pro Leu Ile Ile Asn Arg Ile Gly Gly
            275                 280                 285

Lys Asn Ala Leu Leu Leu Ala Gly Thr Ile Met Ser Val Arg Ile Ile
    290                 295                 300

Gly Ser Ser Phe Ala Thr Ser Ala Leu Glu Val Val Ile Leu Glu Thr
305                 310                 315                 320

Leu His Met Phe Glu Val Pro Phe Leu Leu Val Gly Cys Phe Lys Tyr
                325                 330                 335

Ile Thr Ser Gln Phe Glu Val Arg Phe Ser Ala Thr Ile Tyr Leu Val
            340                 345                 350

Cys Phe Cys Phe Phe Lys Gln Leu Ala Met Ile Phe Met Ser Val Leu
            355                 360                 365

Ala Gly Asn Met Tyr Glu Ser Ile Gly Phe Gln Gly Ala Tyr Leu Val
    370                 375                 380

Leu Gly Leu Val Ala Leu Gly Phe Thr Leu Ile Ser Val Phe Thr Leu
385                 390                 395                 400

Ser Gly Pro Gly Pro Leu Ser Leu Leu Arg Arg Gln Val Asn Glu Val
                405                 410                 415

Ala

## Claims

1. A microorganism which has a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3] below and
which has a higher ability of producing a lactose-containing oligosaccharide than a parent strain,
[1] a protein consisting of the amino acid sequence represented by SEQ ID NO:2,
[2] a mutant protein which is a protein consisting of an amino acid sequence having deletion, replacement, insertion and/or addition of 1 to 20 amino acids in the amino acid sequence represented by SEQ ID NO:2 and which has lactose permease activity, and
[3] a homologous protein which is a protein consisting of an amino acid sequence having at least 80% identity with the amino acid sequence represented by SEQ ID NO:2 and which has lactose permease activity.

2. The microorganism according to claim 1 which has a protein consisting of an amino acid sequence containing replacement of the amino acid residue corresponding to position 319 of the amino acid sequence represented by SEQ ID NO:2 with L-aspartic acid or L-glutamic acid in the amino acid sequence of the protein described in any one of [1] to [3].

3. The microorganism according to claim 1 or 2 which has a protein consisting of an amino acid sequence containing replacement of the amino acid residue corresponding to position 319 of the amino acid sequence represented by SEQ ID NO:2 with L-glutamic acid in the amino acid sequence of the protein described in any one of [1] to [3].

4. The microorganism according to any one of claims 1 to 3 which is obtainable by transforming a parent strain with a recombinant DNA containing a DNA encoding a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3].

5. The microorganism according to claim 4 which is obtainable by incorporating the recombinant DNA containing a DNA encoding a protein consisting of an amino acid sequence containing replacement of the amino acid residue corresponding to position 319 of the amino acid sequence represented by SEQ ID NO:2 with a different amino acid residue in the amino acid sequence of the protein described in any one of [1] to [3] into the chromosome.

6. The microorganism according to any one of claims 1 to 5 which is obtainable by transforming a parent strain with a recombinant DNA containing a DNA having the nucleotide sequence represented by SEQ ID NO:3.

7. The microorganism according to claim 6 which is obtainable by incorporating the recombinant DNA containing a DNA having the nucleotide sequence represented by SEQ ID NO:3 into the chromosome.

8. The microorganism according to any one of claims 1 to 7, wherein the parent strain is a microorganism having an ability of producing a lactose-containing oligosaccharide.

9. A method for producing a lactose-containing oligosaccharide, comprising: culturing the microorganism according to any one of claims 1 to 8 in a medium and producing a lactose-containing oligosaccharide in a culture.

10. The production method according to claim 9, wherein the lactose-containing oligosaccharide is 2'-fucosyllactose.
